# EUROPÄISCHE PATENTSCHRIFT

(11) **EP 1 297 790 B1**
(45) Veröffentlichungstag und Bekanntmachung des Hinweises auf die Patenterteilung: **20.10.2004**
(21) Anmeldenummer: 02020708.0
(22) Anmeldetag: 14.09.2002
(51) Int. Cl.: A61B 17/17

(54) **Chirurgisches Zielgerät**
Surgical drillguide
Guide de perçage chirurgical

(30) Priorität: 20.09.2001 DE 10146452
(43) Veröffentlichungstag der Anmeldung: 02.04.2003
(73) Patentinhaber: Richard Wolf GmbH, 75438 Knittlingen (DE)
(72) Erfinder: Göbel, Jürgen, 76684 Östringen (DE); Körner, Eberhard, 75015 Bretten (DE); Heckele, Helmut, 75438 Knittlingen (DE)
(74) Vertreter: Vollmann, Heiko, Dipl.-Ing.

(56) Entgegenhaltungen:
- US-A- 5 891 150
- US-A- 5 968 050

## Beschreibung

Die Erfindung betrifft ein Zielgerät zum Positionieren eines Bohrwerkzeugs in Bezug auf einen zu schaffenden Bohrkanal, bestehend aus einem Zielhaken und einem lösbar mit diesem verbundenen Führungsarm , an dem eine Aufnahme für das Bohrwerkzeug angeordnet und eine Führungsbahn in Form einer seitlich durchgehend offenen Nut für den Führungsarm ausgebildet ist, der in der Führungsbahn verschiebbar und feststellbar ist

Solche Zielgeräte kommen bei der Rekonstruktion eines beispielweise aufgrund eines Unfalls gerissenen Bandes im Kniegelenk zur Anwendung. Insbesondere besteht für den Ersatz des vorderen Kreuzbandes mit der Quadrizepssehne mittels arthroskopischer Operation die Möglichkeit der implantatfreien Verankerung eines Teils der Quadrizepssehne. Im Rahmen dieser Operation ist es erforderlich, einen spongiösen Knochenzylinder aus der Tibia zu gewinnen, mit dem die Defektauffüllung an der Patella und die zusätzliche Transplantatverblockung in der Tibia vorgenommen werden kann. Hierzu muss zunächst ein Bohrkanal in Tibia und Femur exakt platziert geschaffen werden.

Zur möglichst genauen Platzierung von Bohrkanälen werden Zielgeräte der eingangs erwähnten Art eingesetzt. Aus der US 5 968 050 ist ein solches Zielgerät bekannt. Dieses weist einen Führungsarm auf, an dem über eine entsprechende Aufnahme das Bohrwerkzeug angebracht werden kann. Zur Ausrichtung des Bohrwerkzeugs ist ein Zielhaken mit dem Führungsarm verbunden, der in einem vorab einstellbaren Winkel zum Führungsarm ausgerichtet werden kann. Der Zielhaken weist an seinem distalen Bereich eine gebogene Form mit einer scharfen Spitze auf, die dazu dient, das Ende des Zielhakens während des Bohrvorgangs am Knochen zu verankern, so dass das Zielgerät nicht abrutschen kann. Ähnliche Ausgestaltungen von Zielgeräten sind aus der US 4 672 957, 5 154720 und 5 350 3 83 und aus der EP 0 797 955 A1 bekannt.

Ein Nachteil dieser Zielgeräte besteht darin, dass der in die Gelenkhöhle einzuführende gebogene Zielhaken aufgrund der scharfen Spitze Verletzungen in der Gelenkhöhle verursachen kann. Dieses Risiko kann zwar durch besonders sachgemäße Handhabung des Geräts und bei sorgfältiger Durchführung des endoskopischen Eingriffs gering gehalten, jedoch nie völlig ausgeschlossen werden.

Aufgrund der gebogenen Form des Zielhakens besteht weiterhin der Nachteil, dass eine exakte Positionierung der in den Knochen einzubringenden Bohrung wegen des stets vorhandenen Versatzes zwischen der Spitze des Zielhakens und der sich im Abstand hierzu befindlichen Durchgangsbohrung für den erforderlichen Führungsdraht und den Bohrer nicht möglich ist.

Schließlich ist es bei bekannten Zielgeräten auch nachteilig, dass sie konstruktionsbedingt und aufgrund der anatomischen Gegebenheiten nach erfolgtem Einbringen der Bohrung nur sehr schwer wieder aus der Gelenkhöhle entfernt werden können, zumal eine vorausgehendes Trennen des Führungsarms vom Zielhaken wegen der beengten Platzverhältnisse nicht möglich ist. Deshalb besteht die Gefahr, dass Verletzungen auch in dieser Operationsphase auftreten können.

Der Erfindung liegt die Aufgabe zugrunde, ein Zielgerät der beschriebenen Art so zu verbessern, dass die Gefahr ausgeschlossen wird, dass eine unerwünschte Verletzung der Gelenkhöhle stattfindet, und zwar sowohl beim Einbringen und Anlegen des Endes des Zielhakens in der Gelenkhöhle bzw. am Knochen als auch beim Entfernen des Zielhakens aus der Gelenkhöhle. Ferner soll es möglich werden, das Zielgerät exakt zu positionieren, so dass die Bohrung mit hoher Präzision in den Knochen eingebracht werden kann. Insbesondere soll es möglich sein, im Falle einer Kreuzband-Rekonstruktion den tibialen Ansatzpunkt so genau wie möglich zu setzen.

Diese Aufgabe wird ausgehend von einem Zielgerät der gattungsgemäßen Art so gelöst, dass der Führungsarm von der einen Geräteseite her durch die Nutöffnung in die Führungsbahn eingesetzt werden kann. Hierdurch wird der besondere Vorteil erzielt, dass der Ziel-haken und der Führungsarm einfach durch seitlichen Versatz beider Teile wieder voneinander getrennt werden können, ohne dass es wie bei bekannten Zielgeräten erforderlich ist, zu diesem Zweck den Führungsarm aus der Führungsbahn bzw. Nut herausschieben zu müssen. Für die Verbindung des Zielhakens mit dem Führungsarm gilt umgekehrt gleiches, da der Führungsarm quasi als Einsatz nur von der Seite her in die Führungsbahn eingesetzt werden muß und dann in entsprechender Position festgestellt werden kann. Besonders günstig und einfach wird die Montage und Demontage des Zielgeräts, wenn die Querschnitte der Nut und des Führungsarms jeweils komplementär rechteckig sind.

Am Zielhaken ist ein Sperrknebel mit einem Spannexzenter vorgesehen, mit dem der Führungsarm durch Verspannen in der Führungsbahn bzw. Nut festgelegt werden kann, und zwar durch Verdrehen des Sperrknebels in eine Richtung. Durch Verdrehen des Sperrknebels in entgegengesetzte Richtung kann die kraftschlüssige Verbindung wieder gelöst und der Führungsarm einfach vom Zielhaken aus dessen Führungsbahn heraus abgehoben werden.

Das distale freie Ende des Zielhakens ist durch ein ringförmiges Durchgangsteit mit einer Durchgangsöffnung für das Bohrwerkzeug gebildet, wobei die Achsen für die Aufnahme des Bohrwerkzeugs und die Durchgangsöffnung fluchten. Weiterhin ist am distalen Ende des Zielhakens eine proximalwärts zur Aufnahme hin gerichtete Schneide als Ersatz für die sonst übliche scharfe Spitze vorgesehen. Diese Schneide ist zweckmäßigerweise am proximalen und zur Aufnahme hin gerichteten Ende des Durchgangsteils angeordnet.

Um den Führungsdraht für das Bohrwerkzeug und das Bohrwerkzeug selbst leicht in und durch die Durchgangsöffnung bringen zu können und das Bohrwerkzeug schließlich axial in der Durchgangsöffnung einwandfrei zu führen, weist diese in einem ersten distalen Abschnitt eine zylindrische Kontur auf, an die sich in einem zweiten Abschnitt eine sich proximalwärts erweiternde kegelstumpfförmige Kontur anschließt, so dass der Führungsdraht leicht in den ersten Abschnitt eingeführt werden kann und schließlich das Bohrwerkzeug im distalen Abschnitt geführt wird.

Mit dem erfindungsgemäßen Zielgerät ist nicht nur eine atraumatische und damit schonende Positionierung des Endbereichs des Zielhakens in der Gelenkhöhle möglich, sondern auch die einfache Entfernung des Zielhakens aus diesem Bereich, nachdem vorab Führungsarm und Zielhaken wie beschrieben voneinander getrennt wurden, so dass der Operateur beim Entfernen des Zielhakens nicht durch den Führungsarm behindert wird und den Zielhaken kontrolliert aus der Gelenkhölle entfernen kann. Außerdem kann eine präzise Platzierung der Bohrung erfolgen, da der Endbereich des Zielhakens mit geringem radialen und axialen Abstand an der Insertionsstelle anliegt. Es wird damit insbesondere ermöglicht, dass auch hierdurch nach gesetzter Zielbohrung der Zielhaken unabhängig vom entfernten Führungsarm an Ort und Stelle entnommen werden kann.

In der Zeichnung ist ein Ausführungsbeispiel der Erfindung dargestellt. Es zeigen:
- Fig. 1: eine perspektivische Ansicht eines Zielgeräts ohne eingesetztes Bohrwerkzeug,
- Fig. 2: den vom Zielgerät abgenommenen Zielhaken in perspektivischer Ansicht,
- Fig. 3: eine zu Fig. 1 analoge Darstellung, wobei das Zielgerät jedoch um 180° geschwenkt wurde,
- Fig. 4: das Zielgerät mit eingesetztem Bohrwerkzeug in einer zum bearbeiteten Knochen ausgerichteten Position,
- Fig. 5: den Endabschnitt des Zielhakens in perspektivischer Ansicht und
- Fig. 6: den Schnitt A-A gemäß Fig. 5.

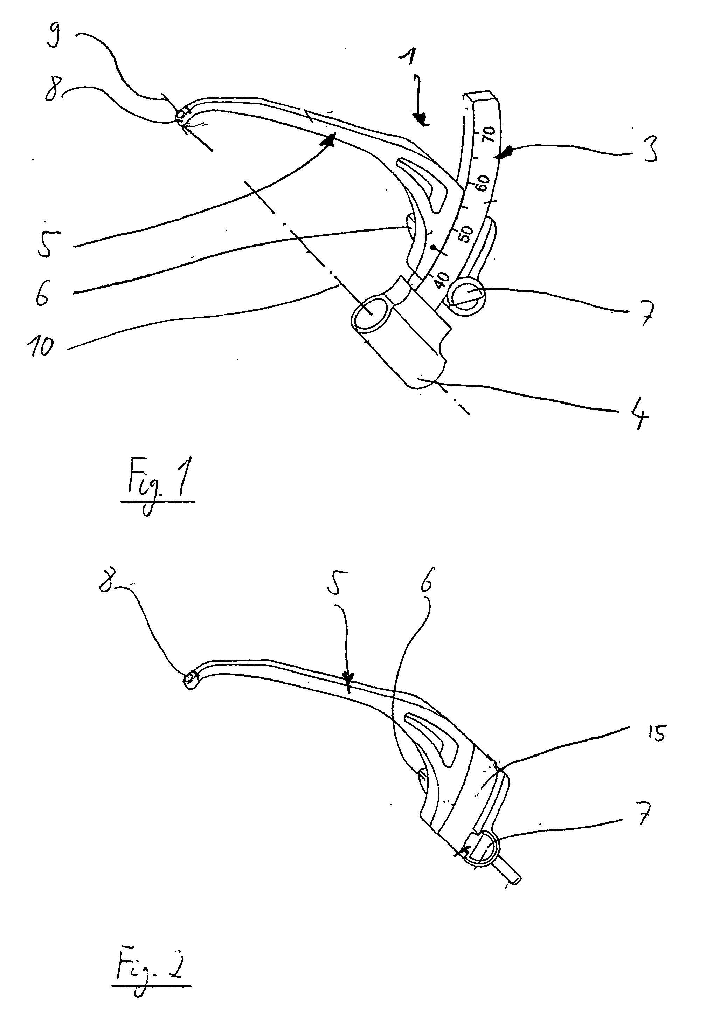

In den Fig. 1, 3 und 4 ist ein Zielgerät 1 zu sehen, das im Wesentlichen aus einem Führungsarm 3 und einem daran befestigten Zielhaken 5 besteht. Der Führungsarm 3 hat eine Aufnahme 4, die eine hohlzylindrische Gestalt hat und in die das Gehäuse eines Bohrwerkzeugs 2 eingesetzt werden kann.

Das Bohrwerkzeug 2 wird während des Einbringens einer Bohrung in einen Knochen (Fig. 4) axial in Richtung auf den Knochen verstellt. Dazu ist, was nicht dargestellt ist, am zylindrischen Gehäuse des Bohrwerkzeugs 2 zumindest über einen Teil des Umfangs, vorzugsweise über den halben Umfang, eine Verzahnung angeordnet, die mit einer in der Aufnahme 4 angeordneten Sperrklinke zusammenwirkt. Die Sperrklinke ist mit einer Druckfeder vorgespannt. Damit kann das Bohrwerkzeug stufenweise distalwärts in der Aufnahme 4 verschoben werden, wobei ein Rückstellen des Werkzeugs dadurch verhindert wird, dass die Verzahnung in Rückstellrichtung senkrechte Flanken aufweist, an denen die Sperrklinke sperrend zur Anlage gelangen wird. Es ist also im Betrieb des Bohrwerkzeugs nur eine axiale Verschiebung des Bohrers in Richtung auf das distale Ende des Zielhakens möglich, nicht jedoch in proximalwärtiger Richtung. Zum Entfernen des Bohrwerkzeugs kann der beschriebene Sperrmechanismus von Hand deaktiviert werden, so dass das Bohrwerkzeug samt Bohrer vom Zielgerät abgezogen werden kann.

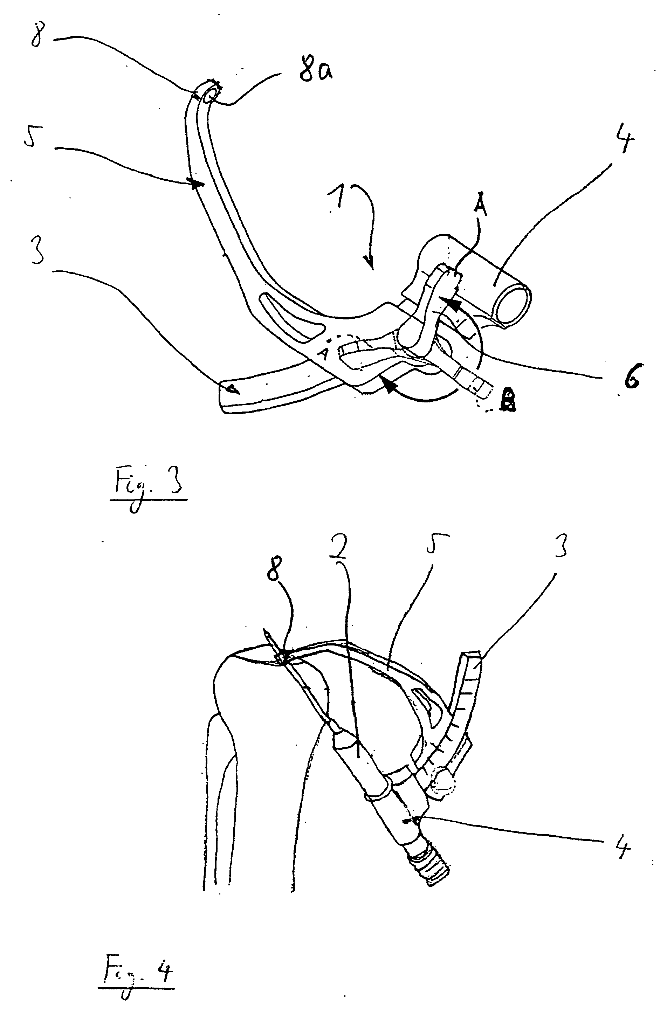

Der Führungsarm 3 des Zielgeräts 1 ist bogenförmig ausgebildet und weist eine Winkelskala auf, die angibt, unter welchem Winkel der Zielhaken 5 zur Achse 10 des Bohrwerkzeugs 2 eingestellt ist. Entsprechend der Form des Bogens des Führungsarms 3 ist in den Zielhaken 5 eine komplementäre, bogenförmige Führungsbahn 15 in Form einer Nut (Fig.2) eingearbeitet. An einem Ende der Führungsbahn 15 ist ein Spannexzenter 7 angeordnet, der über einen drehbaren Sperrknebel 6 verstellt werden kann.

Wenn sich der Spannexzenter 7 in der in Fig. 2 dargestellten Position befindet, kann der Zielhaken 5 auf dem Führungsarm 3 verschoben oder von diesem einfach von Hand abgehoben werden. Wird der Sperrknebel 6 um einen bestimmten Winkel verschwenkt, dann wird der Zielhaken 5 aufgrund des vom Spannexzenter 7 auf den Führungsarm 3 ausgeübten Drucks der Führungsbahn 15 festgelegt.

Hierzu kann der Sperrknebel 6, wie es Fig. 3 zu sehen ist, wahlweise im Uhrzeigersinn oder entgegen diesem Drehsinn gedreht werden. In Fig. 3 ist der Sperrknebel 6 mit ausgezogenen Linien in einer Position A dargestellt, bei der der Führungsarm 3 und Zielhaken 5 fest miteinander durch Kraftschluss verbunden sind. In der Spannstellung A liegt der Sperrknebel 6 gemäß Fig. 3 so,dass bei der Operation kein versehentliches Verdrehen des Sperrknebels 6 möglich ist. Die Position des Sperrknebels 6, in welcher der Zielhaken 5 vom Führungsarm 3 durch Abheben abgenommen werden kann, ist in Fig. 3 mit B bezeichnet. In Fig. 4 ist zu sehen, wie das distalseitige Ende des Zielhakens 5 mit einer später noch beschriebenen Schneide an der Knochenoberfläche anliegt.

Zum präzisen Festlegen des mittels des Bohrwerkzeugs 2 in den Knochen einzubringenden Bohrkanals weist der Zielhaken 5 an seinem distalen Ende ein ringförmiges oder zylinderförmiges Durchgangsteil 8 auf, das in das Ende des Zielhakens 5 eingeformt ist. Das Durchgangsteil 8, das äußerlich über den halben Umfang im Wesentlichen die Form eines Zylinders aufweist - über den restlichen Umfang ist es mit dem Ende des Zielhakens 5 integral verbunden - hat eine Durchgangsöffnung 8a für das Bohrwerkzeug mit einer Achse 9, die mit der Achse 10 des Bohrwerkzeugs 2 fluchtet (Fig. 1).

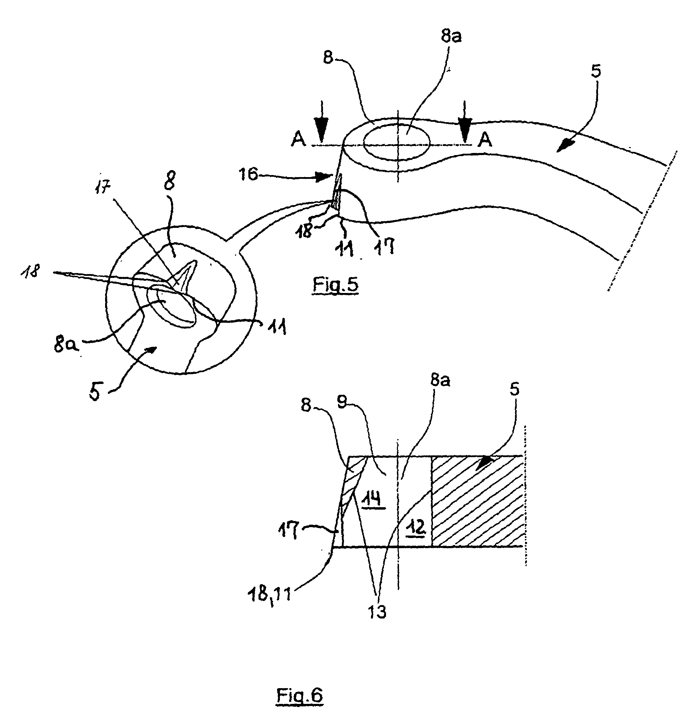

Details der Ausgestaltung des distalen Zielhakenendes ergeben sich aus den Fig. 5 und 6. Während die Außenkontur des Durchgangsteils 8 weitgehend zylindrisch ist, besteht der innere Durchgang dieses Teils aus einem halbkegelstumpfförmigen Abschnitt 14 und einem halbzylinderförmigen Abschnitt 12, deren Mantelflächen 13 fließend ineinander übergehen.

Im distalen freien Ende des Zielhakens 5 ist eine proximalwärts zur Aufnahme 4 hin gerichtete Schneide 11 vorgesehen, die am proximalen Ende des Durchgangsteils 8 ausgebildet ist (Fig. 6). Diese Schneide dient zur sicheren Anlage des distalen Zielhakenendes am Knochen. Aus der Fig. 6 ist ersichtlich, dass der freie und wirksame Abschnitt der Schneide nur auf einem Teil eines Kreises verläuft.

Um ein Abgleiten der Schneide 11 bzw. Schneidkante vom Knochen besonders wirksam zu verhindern, ist die Schneide durch eine beispielsweise V-förmige Ausnehmung 17 geteilt, wodurch zwei im geringen Abstand zueinander angeordnete hakenförmige scharfe Kanten 18 entstehen.

Im Vergleich mit bekannten Ausgestaltungen des distalen Zielhakenendes mit einer Hakenspitze ergibt sich, dass bei der erfindungsgemäßen Ausführung nur ein kleiner radialer Abstand zwischen dem Anlagepunkt der Schneide 11 am Knochen und der Achse 10 des Bohrwerkzeugs 2 gegeben ist. Dies ermöglicht eine wesentlich exaktere Positionierung der Bohrung als mit herkömmlichen Zielgeräten.

Die Form des Endbereichs 16 des ringförmigen Teils 8 mit der Schneide 11 ermöglicht ein Einhaken des distalen Endes des Zielhakens 5 direkt an der tibialen Ansatzstelle, z. B. des vorderen Kreuzbandes. Durch das ringförmige, außen abgerundete Durchgangsteil 8 am distalen Ende des Zielhakens 5 ergibt sich der Vorteil, dass der Zielhaken atraumatisch in das Kniegelenk eingeführt und auch wieder aus diesem entfernt werden kann, ohne dass es dabei zu Verletzungen von Gewebe durch die Schneide kommen kann.

## Patentansprüche

1. Zielgerät (1) zum Positionieren eines Bohrwerkzeugs (2) in Bezug auf einen zu schaffenden Bohrkanal, bestehend aus einem Zielhaken (5) und einem lösbar mit diesem verbundenen Führungsarm (3), an dem eine Aufnahme (4) für das Bohrwerkzeug (2) angeordnet und eine Führungsbahn (15) in Form einer seitlich durchgehend offenen Nut für den Führungsarm (3) ausgebildet ist, der in der Führungsbahn (15) verschiebbar und feststellbar ist, **dadurch gekennzeichnet, dass** der Führungsarm (3) von der einen Geräteseite her durch die Nutöffnung in die Führungsbahn (15) einsetzbar ist.

2. Zielgerät nach Anspruch 1, **dadurch gekennzeichnet, dass** die Querschnitte der Nut (15) und des Führungsarms (3) komplementär zueinander ausgebildet sind.

3. Zielgerät nach einem der Ansprüche 1 oder 2, **dadurch gekennzeichnet, dass** am Zielhaken (5) ein Sperrknebel (6) mit einem Spannexzenter (7) vorgesehen ist, mit dem der Führungsarm (3) durch Verspannen in der Führungsbahn (15) feststellbar ist.

4. Zielgerät nach einem der Ansprüche 1 bis 3, **dadurch gekennzeichnet, dass** das distale freie Ende des Zielhakens (5) durch ein ringförmiges Durchgangsteil (8) mit einer Durchgangsöffnung (8a) für das Bohrwerkzeug (2) gebildet ist und dass die Achsen (9, 10) der Aufnahme (4) und der Durchgangsöffnung fluchten.

5. Zielgerät nach einem der Ansprüche 1 bis 4, **dadurch gekennzeichnet, dass** am distalen freien Ende des Zielhakens (5) eine proximalwärts zur Aufnahme (5) hin gerichtete Schneide (11) vorgesehen ist.

6. Zielgerät nach einem der Ansprüche 4 und 5, **dadurch gekennzeichnet, dass** die Durchgangsöffnung (8a)in einem ersten Abschnitt (12) eine zylindrische Kontur aufweist, an die sich in einem zweiten Abschnitt (14) eine sich proximalwärts erweiterende kegelstumpfförmige Kontur anschießt.

7. Zielgerät nach einem der Ansprüche 5 und 6, **dadurch gekennzeichnet, dass** die Schneide (11) am proximalen, zur Aufnahme (4) hin gerichteten Ende des Durchgangsteils (8) angeordnet ist.

8. Zielgerät nach einem der Ansprüche 5 bis 7, **dadurch gekennzeichnet, dass** die Schneide (11) durch eine Ausnehmung (17) derart geteilt ist, dass zwei im Abstand zueinander angeordnete hakenförmige scharfe Kanten (18) entstehen.

## Claims

1. A drillguide (1) for positioning a drilling tool (2) with respect to a drill channel to be created, consisting of a targeting hook (5) and a guide arm (3) detachably connected to this, on which drillguide there is arranged a receiver (4) for the drilling tool (2), and on which there being formed a guide path (15) in the form of a laterally continuously open groove for the guide arm (3), said guide arm being displaceable and fixable in the guide path (15), **characterised in that** the guide arm (3) from the one side of the apparatus may be inserted through the groove opening into the guide path (15).

2. A drillguide according to claim 1, **characterised in that** the cross section of the groove (15) and of the guide arm (3) are formed complementarily to one another.

3. A drillguide according to one of the claims 1 or 2, **characterised in that** on the targeting hook (5) there is provided a locking toggle (6) with a clamping eccentric (7) with which the guide arm (3) may be fixed by way of clamping in the guide path (15).

4. A drillguide according to one of the claims 1 to 3, **characterised in that** the distal free end of the targeting hook (5) is formed by an annular passage part (8) with a passage opening (8a) for the drilling tool (2) and that the axes (9, 10) of the receiver (4) and of the passage opening are aligned.

5. A drillguide according to one of the claims 1 to 4, **characterised in that** at the distal free end of the targeting hook (5) there is provided a cutter (11) directed proximally towards the receiver (4).

6. A drillguide according to one of the claims 4 and 5, **characterised in that** the passage opening (8a) in a first section (12) has a cylindrical contour to which, in a second section (14), a proximally widening truncated cone-shaped contour connects.

7. A drillguide according to one of claims 5 and 6, **characterised in that** the cutter (11) is arranged at the proximal end of the passage part (8), said end being directed towards the receiver (4).

8. A drillguide according to one of the claims 5 to 7, **characterised in that** the cutter (11) is divided by a recess in a manner such that there arise two hook-like sharp edges (18) arranged at a distance to one another.

## Revendications

1. Appareil de visée (1) pour positionner un outil de perçage (2) relativement à un canal de perçage à réaliser, comprenant un crochet de visée (5) et, relié de manière amovible à celui-ci, un bras de guidage (3) dans lequel est disposé un logement de réception (4) pour un outil de perçage (2), une glissière de guidage (15), sous la forme d'une rainure ouverte latéralement en continu, étant réalisée dans le crochet de visée pour le bras de guidage (3) qui peut coulisser et être immobilisé dans la glissière de guidage (15),
**caractérisé en ce que** le bras de guidage (3) peut être inséré dans la glissière de guidage (15) à partir du côté latéral considéré de l'appareil, à travers l'ouverture de la rainure.

2. Appareil de visée selon la revendication 1, **caractérisé en ce que** les sections transversales de la rainure (15) et du bras de guidage (3) sont réalisées de manière à être mutuellement complémentaires.

3. Appareil de visée selon l'une des revendications 1 ou 2, **caractérisé en ce que** sur le crochet de visée (5) est prévue une manette de blocage (6) comprenant un excentrique de serrage (7), à l'aide desquels le bras de guidage (3) peut être immobilisé par serrage dans la glissière de guidage (15).

4. Appareil de visée selon l'une des revendications 1 à 3, **caractérisé en ce que** l'extrémité libre distale du crochet de visée (5) est formée par une pièce de passage (8) de forme annulaire, comportant une ouverture de passage (8a) pour l'outil de perçage (2), et **en ce que** les axes (9, 10) du logement de réception (4) et de l'ouverture de passage sont mutuellement alignés.

5. Appareil de visée selon l'une des revendications 1 à 4, **caractérisé en ce qu'**à l'extrémité libre distale du crochet de visée (5), est prévu un tranchant de coupe (11) orienté en direction proximale vers le logement de réception (4).

6. Appareil de visée selon l'une des revendications 4 et 5, **caractérisé en ce que** l'ouverture de passage (8a) présente, sur un premier secteur (12), un contour cylindrique auquel se raccorde, dans un second secteur (14), un contour de forme tronconique s'élargissant en direction proximale.

7. Appareil de visée selon l'une des revendications 5 et 6, **caractérisé en ce que** le tranchant de coupe (11) est disposé à l'extrémité proximale de la pièce de passage (8), extrémité dirigée vers le logement de réception (4).

8. Appareil de visée selon l'une des revendications 5 à 7, **caractérisé en ce que** le tranchant de coupe (11) est divisé par une encoche (17) de façon telle que soient formées deux arêtes vives (18) en forme de crochet, disposées à distance l'une de l'autre.
